# EUROPEAN PATENT APPLICATION

(11) **EP 4 159 119 A1**
(43) Date of publication of application: **05.04.2023**
(21) Application number: 21889126.5
(22) Date of filing: 29.10.2021
(51) Int. Cl.: A61B 5/026, A61B 5/0245

(54) **NON-CONTACT BLOOD VESSEL ANALYZER**

(30) Priority: 04.11.2020 JP 2020184732
(71) Applicant: Leimac Ltd., Shiga 5240215 (JP)
(72) Inventor: SHIMAZAKI, Takunori, Kawanishi-shi Hyogo 666-0101 (JP); KAWAKUBO, Yoshifumi, Awa-shi, Tokushima 771-1507 (JP); MITSUDO, Jun, Moriyama-shi Shiga 524-0215 (JP); HAYASHI, Yuhei, Moriyama-shi Shiga 524-0215 (JP); HATABE, Shinya, Moriyama-shi Shiga 524-0215 (JP)
(74) Representative: Zimmermann, Tankred Klaus
(86) International application number: PCT/JP2021/039942
(87) International publication number: WO 2022/097573

(57) **Abstract**

Provided is a non-contact blood vessel analyzer capable of easily analyzing the state of a blood vessel in a non-contact manner without limiting a place. This non-contact blood vessel analyzer 1 includes an image acquisition device 2 which acquires an image 2im which is a moving image or successive still images of a blood vessel, and an image processing device 3 which detects a beat and a thrill from temporal change of brightness or luminance of the image 2im. The image processing device 3 can detect, as the beat, a largest value vicinity area in a power spectrum of the brightness or the luminance of the image 2im, use a frequency at which power value is the largest in the power spectrum of the brightness or the luminance of the image 2im as a heart rate, and detect, as the thrill, a maximum value vicinity area when a maximum value increases temporarily after the maximum value decreases as the frequency increases in the power spectrum of the brightness or the luminance of the image 2im.

## Description

### NON-CONTACT BLOOD VESSEL ANALYZER

### [Technical Field]

The present invention relates to a non-contact blood vessel analyzer capable of analyzing the state of a blood vessel influenced by anastomosis in a non-contact manner.

### [Background Art]

In treatment such as dialysis in which blood of a patient is circulated extracorporeally through an extracorporeal circulation blood circuit, an inlet/outlet part (vascular access) for taking blood from a patient or returning the blood to the patient is provided. In the vascular access, there is used a method in which, for the extracorporeal circulation, an artery and a vein are anastomosed to each other directly by a surgical operation or by using an artificial blood vessel such that arterial blood is caused to flow directly into a vein, and an arteriovenous anastomosis part is thereby formed.

However, by forming the arteriovenous anastomosis part, a blood vessel influenced by the formation of the arteriovenous anastomosis part is narrowed and further blocked due to thickening and hardening of an intima of the blood vessel and blood clot formation, and a blood circulation disorder easily occurs. To cope with this, it is determined whether or not the blood vessel has an abnormality before and after the treatment by listening to sound or feeling movement of a pulse. In a method of listening to sound, typically, it is determined whether or not the abnormality is present by listening to the volume and range of a blood flow sound (what is called a shunt sound) with a stethoscope. That is, while it is possible to listen to a loud low-pitched sound when a blood vessel is normal, the volume of the low-pitched sound is reduced and the volume of a high-pitched sound is increased when the blood vessel is narrowed. When the narrowing further progresses, the volume of the sound is reduced, and it becomes impossible to listen to the sound when the blood vessel is blocked.

In addition, in a method of feeling the movement of the pulse, typically, it is determined whether or not the abnormality is present by putting a finger on a blood vessel and feeling vibration (thrill) caused by a turbulent flow generated when blood of an artery having high pressure is caused to flow into a vein which impinges on a blood vessel wall. That is, it is possible to feel the thrill when the blood vessel is normal. But, when the blood vessel is narrowed and is about to be blocked, a blood flow is reduced, and hence the thrill disappears.

So far, various techniques have been proposed in order to analyze the state of such a blood vessel to easily find the abnormality. For example, Patent Document 1 discloses a technique for acquiring a shunt sound with a shunt sound acquisition device provided in an extracorporeal circulation blood circuit, converting the shunt sound to an electrical signal, and analyzing pulsation and the like. In addition, Patent Document 2 discloses a technique for acquiring a shunt sound with a shunt sound analyzer, converting the shunt sound to an electrical signal, extracting characteristic components representing characteristics of the shunt sound caused by narrowing of a blood vessel from the electrical signal, and outputting evaluation information related to evaluation of the shunt sound based on the characteristic components.

### [Prior Art Documents]

### [Patent Documents]

[Patent Document 1] Japanese Patent Application Laid-open No. 2005-328941
[Patent Document 2] WO 2016/207951

### [Summary of Invention]

### [Technical Problem]

However, in the analyzer which uses the shunt sound as in each of Patent Document 1 and Patent Document 2, quiet environment or measures to shield external noise is required in order to block external noise, and hence a place where the analyzer can be used is limited. Further, it is necessary to cause a microphone or the like to come into contact with a skin, and hence measures against infection caused by the contact is required.

The present invention has been made in view of such reasons, and an object thereof is to provide a non-contact blood vessel analyzer capable of easily analyzing the state of a blood vessel in a non-contact manner without being limited to a specific place.

### [Solution to Problem]

In order to attain the above object, a non-contact blood vessel analyzer according to an embodiment of the present invention includes an image acquisition device which acquires an image which is a moving image or successive still images of a blood vessel, and an image processing device which detects a beat and a thrill from temporal change of an index derived from brightness and / or chromaticity of the image.

Preferably, the non-contact blood vessel analyzer further includes a light irradiation device which irradiates the blood vessel with light.

Preferably, the index derived from the brightness and / or the chromaticity of the image is brightness or luminance of the image.

Preferably, the image processing device detects, as the beat, a largest value vicinity area in a power spectrum of the brightness or the luminance of the image.

Preferably, the image processing device uses a frequency at which power value is the largest in the power spectrum of the brightness or the luminance of the image as a heart rate.

Preferably, the image processing device detects, as the thrill, a maximum value vicinity area when a maximum value increases temporarily after the maximum value decreases as the frequency increases in the power spectrum of the brightness or the luminance of the image.

According to the non-contact blood vessel analyzer according to the present invention, it becomes possible to easily analyze the state of the blood vessel in a non-contact manner without limiting the non-contact blood vessel analyzer to a specific place.

### [Brief Description of Drawings]

[Fig. 1]

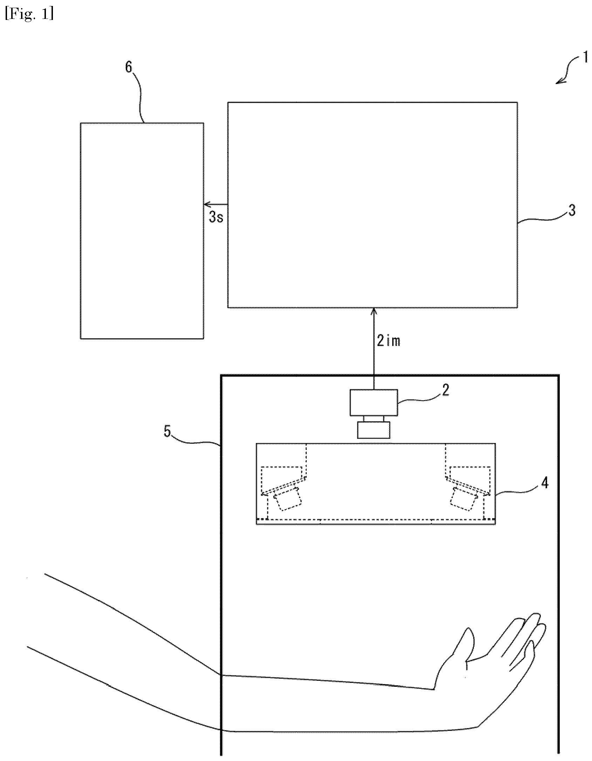

   Fig. 1 is a schematic view showing an example of use of a non-contact blood vessel analyzer according to an embodiment of the present invention.
[Fig. 2]

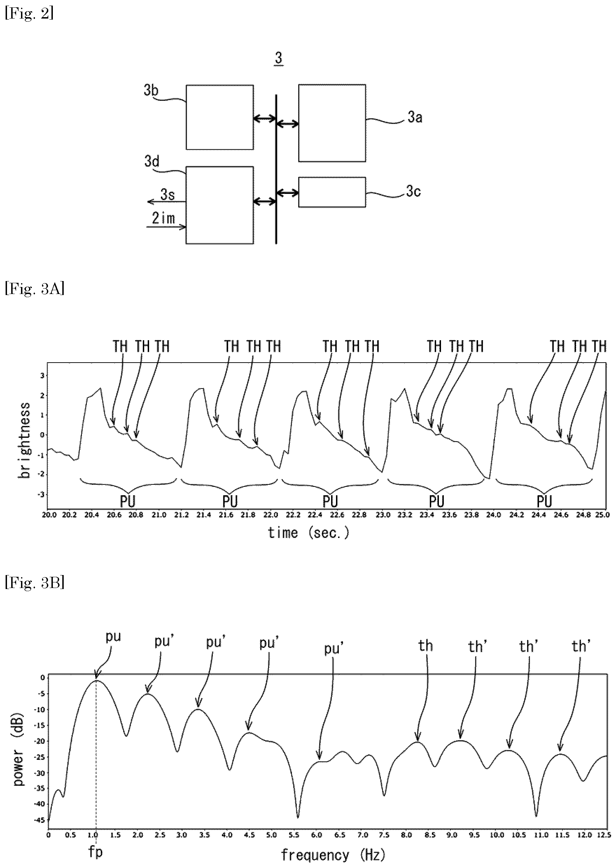

   Fig. 2 is a schematic view showing an image processing device implemented by a computer system in the non-contact blood vessel analyzer shown in Fig. 1.
[Fig. 3A]
   Fig. 3A shows an example of analysis in the example of use shown in Fig. 1, and is a graph of data representing temporal change of brightness of an image acquired in an arteriovenous anastomosis part.
[Fig. 3B]
   Fig. 3B shows an example of analysis in the example of use shown in Fig. 1, and is a graph (the vertical axis indicates power and the horizontal axis indicates frequency) of a power spectrum of the data shown in Fig. 3A.
[Fig. 4A]

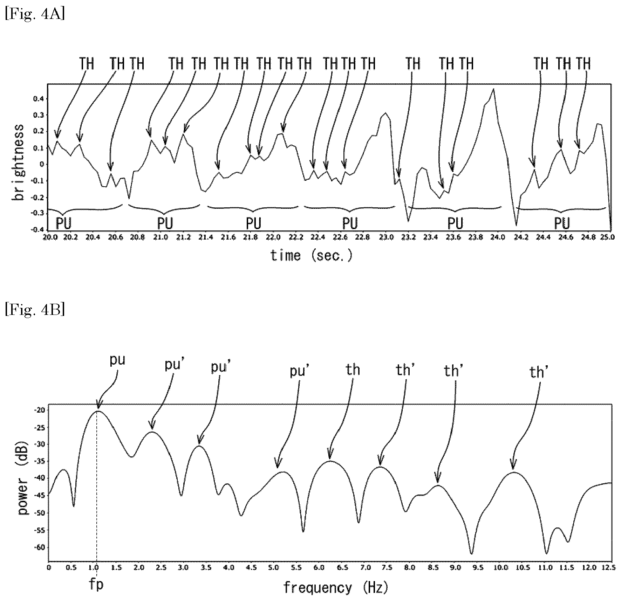

   Fig. 4A shows an example of analysis in the example of use shown in Fig. 1, and is a graph of data representing temporal change of brightness of an image acquired in a portion positioned away from the arteriovenous anastomosis part.
[Fig. 4B]
   Fig. 4B shows an example of analysis in the example of use shown in Fig. 1, and is a graph (the vertical axis indicates power and the horizontal axis indicates frequency) of a power spectrum of the data shown in Fig. 4A.

### [Description of Embodiments]

Hereinbelow, an embodiment for carrying out the present invention will be described. As shown in Fig. 1, a non-contact blood vessel analyzer 1 according to an embodiment of the present invention includes an image acquisition device 2 and an image processing device 3.

The image acquisition device 2 acquires an image 2im which is a moving image or successive still images by imaging a blood vessel (e.g., an arteriovenous anastomosis part) of a patient.

In order to acquire the image 2im, the non-contact blood vessel analyzer 1 can include, in addition to the image acquisition device 2, a light irradiation device 4 (a ringshaped lighting device in Fig. 1) which irradiates the blood vessel of the patient with light, and a light-blocking box 5 which blocks outside light. Light of the light irradiation device 4 is single-color light (e.g., green light or the like) or white light, and may also be, in addition to visible light, light in an infrared region or an ultraviolet region.

In the blood vessel, the amount of red blood cells fluctuates and absorbance fluctuates due to changes such as contraction and expansion by a beat. When the blood vessel is imaged, it is possible to acquire the image 2im which is a moving image or successive still images in which brightness and / or chromaticity changes with time due to the fluctuation of the absorbance and / or physical change of the blood vessel. When a blood vessel influenced by arteriovenous anastomosis is imaged, it is possible to acquire the image 2im which includes changes caused by a thrill in addition to changes caused by a beat. Note that, in an experiment conducted by the inventors of the present application, the fluctuation of the absorbance to green light was conspicuous.

The image processing device 3 detects the beat and the thrill from temporal change of an index derived from brightness and / or chromaticity of the image 2im acquired by the image acquisition device 2. Herein, the index derived from brightness and / or chromaticity is derived from brightness, a hue, chroma, or a combination thereof, and includes luminance. When it is assumed that the index derived from brightness and / or chromaticity is brightness or luminance, as will be described later, it is possible to easily show the temporal change thereof graphically and easily analyze the temporal change thereof. Hereinafter, when a description is made by using brightness, contents of the description are not changed even when luminance is used instead of brightness.

As shown in Fig. 2, the image processing device 3 is usually implemented by a computer system, and has a program for image processing in a program memory 3a. In Fig. 2, the reference sign 3b denotes a CPU, the reference sign 3c denotes a work memory, and the reference sign 3d denotes other parts including an input-output unit.

When it is assumed that the index derived from the brightness and / or the chromaticity of the image 2im is brightness, as shown in Fig. 3A, change of the brightness includes a small and sharp change TH by a thrill in addition to a large and gentle change PU by a beat. Note that the image 2im used in a graph in Fig. 3A (and Fig. 4A described later) is acquired by irradiating a patient with green light with the light irradiation device 4. In addition, the graph in Fig. 3A (and Fig. 4A described later), extraction is performed in a frequency range of 0.8 Hz to 12 Hz. Further, the graph in Fig. 3A (and Fig. 4A described later) shows data in a period between twenty seconds after start of data collection and twenty-five seconds after the start of data collection.

In the image processing device 3, by performing Fourier transformation on the waveform of the temporal change of the brightness of the image 2im as shown in Fig. 3B and performing frequency analysis (i.e., analyzing a power spectrum of the brightness to frequency), it is possible to detect the beat and the thrill.

For example, it is possible to detect a largest value vicinity area pu of power in a power spectrum of the brightness of the image 2im as the beat, and use a frequency fp at which the power value is the largest as a heart rate. Subsequently, with the intensity of the detected beat or the frequency of the heart rate, it is also possible to evaluate whether or not the beat is normal. In the power spectrum of the brightness to the frequency, in general, among a plurality of maximum value vicinity areas, a maximum value vicinity area having the lowest frequency (fundamental wave) serves as the largest value vicinity area, and indicates the beat. In addition, in the area, a harmonic pu' in which the maximum value gradually decreases as the frequency increases also appears.

In addition, for example, it is possible to detect, as a thrill th, the maximum value vicinity area when the maximum value increases temporarily after the maximum value decreases as the frequency increases in the power spectrum of the brightness of the image 2im. In this case, it is possible to regard the maximum value vicinity area before the maximum value vicinity area detected as the thrill as the above-mentioned harmonic pu'. Further, it is also possible to evaluate whether or not the thrill is normal with the intensity of the detected thrill or the presence or absence of the thrill. In addition, in Fig. 3B, a thrill th' appears in addition to the thrill denoted by the reference sign th.

Note that, in the image processing device 3, as the specific method for detecting the beat and the thrill, various methods can be used besides that. For example, it is also possible to detect the beat and the thrill with deep learning or LiDAR (Light Detection And Ranging) or the like.

Thus, in the non-contact blood vessel analyzer 1, it is possible to easily analyze the state of the blood vessel in a non-contact manner without limiting the place by detecting the beat and the thrill using the image acquisition device 2 and the image processing device 3 instead of using the conventional method of listening to sound and the conventional method of feeling the movement of a pulse. In addition, the non-contact blood vessel analyzer 1 has the simple configuration. Further, the non-contact blood vessel analyzer 1 does not come into contact with a patient, and hence it is possible to perform analysis in a state in which a blood vessel is not influenced by contact.

Note that the non-contact blood vessel analyzer 1 includes a display device 6 (see Fig. 1), and display data 3s from the image processing device 3 is input to the display device 6, and the graphs shown in Fig. 3A and Fig. 3B can be concurrently or selectively displayed on the display device 6.

In addition, imaging is not limited to the arteriovenous anastomosis part, and the imaging can be performed on blood vessels of other portions as long as the blood vessel has a blood flow influenced by anastomosis between an artery and a vein. Even in a portion positioned away from the arteriovenous anastomosis part, the brightness changes with time as shown in Fig. 4A, and it is possible to detect the beat and the thrill as shown in Fig. 4B.

All parts of the non-contact blood vessel analyzer 1 can be unified, and it is possible to use, e.g., a camera-equipped terminal such as a smartphone or a notebook-size personal computer.

While the non-contact blood vessel analyzer according to the embodiment of the present invention has been described thus far, the present invention is not limited to the invention described in the above embodiment, and various design changes can be made within the scope of matters described in the claims.

### [Reference Signs List]

- 1: Non-contact blood vessel analyzer
- 2: Image acquisition device
- 2im: Image
- 3: Image processing device
- 3a: Program memory
- 3b: CPU
- 3c: Work memory
- 3d: Other parts of computer system
- 3s: Display data
- 4: Light irradiation device
- 5: Light-blocking box
- 6: Display device
- fp: Frequency at which the power value is the largest in power spectrum of brightness of image
- PU: Large and gentle change by beat
- pu: Largest value vicinity area of power in power spectrum of brightness of image (fundamental wave)
- pu': Harmonic
- TH: Small and sharp change by thrill
- th, th': thrill

## Claims

1. A non-contact blood vessel analyzer comprising:
an image acquisition device which acquires an image which is a moving image or successive still images of a blood vessel; and
an image processing device which detects a beat and a thrill from temporal change of an index derived from brightness and / or chromaticity of the image.

2. The non-contact blood vessel analyzer according to claim 1, further comprising a light irradiation device which irradiates the blood vessel with light.

3. The non-contact blood vessel analyzer according to claim 1 or 2, wherein the index derived from the brightness and / or the chromaticity of the image is brightness or luminance of the image.

4. The non-contact blood vessel analyzer according to claim 3, wherein the image processing device detects, as the beat, a largest value vicinity area in a power spectrum of the brightness or the luminance of the image.

5. The non-contact blood vessel analyzer according to claim 4, wherein the image processing device uses a frequency at which power value is the largest in the power spectrum of the brightness or the luminance of the image as a heart rate.

6. The non-contact blood vessel analyzer according to any one of claims 3 to 5, wherein the image processing device detects, as the thrill, a maximum value vicinity area when a maximum value increases temporarily after the maximum value decreases as the frequency increases in the power spectrum of the brightness or the luminance of the image.
